# EUROPÄISCHE PATENTANMELDUNG

(11) **EP 4 574 987 A1**
(43) Veröffentlichungstag der Anmeldung: **25.06.2025**
(21) Anmeldenummer: 23220103.8
(22) Anmeldetag: 22.12.2023
(51) Int. Cl.: C12P 17/04

(54) **VERFAHREN ZUR HERSTELLUNG VON 2,5-FURANDICARBONSÄURE**

(71) Anmelder: Annikki GmbH, 8074 Raaba-Grambach (AT); Treemera GmbH, 83064 Raubling (DE)
(72) Erfinder: Staunig, Nicole, 8074 Raaba-Grambach (AT); Dupont, Maria, 8074 Raaba-Grambach (AT)
(74) Vertreter: Schwarz & Partner Patentanwälte GmbH

(57) **Zusammenfassung**

Die vorliegende Erfindung betrifft ein Verfahren zur Herstellung von 2,5-Furandicarbonsäure, indem 5-Formyl-2-furancarbonsäure, welche in einer wässrigen Lösung vorliegt, durch Behandeln mit einer NAD(P)⁺-abhängigen Oxidoreduktase *in vitro* zu 2,5-Furandicarbonsäure oxidiert wird, wobei das bei der Oxidation entstandene NAD(P)H enzymatisch mittels einer NAD(P)H-Oxidase wieder zu NAD(P)⁺ oxidiert wird, wonach die Enzyme entfernt werden.

## Beschreibung

Die Erfindung betrifft ein Verfahren zur Herstellung von 2,5-Furandicarbonsäure (FDCA) aus 5-Formyl-2-furancarbonsäure (FFA).

### Hintergrund der Erfindung

Im Jahr 2018 basierten in etwa 90% der produzierten Kunststoffe weltweit (400 Mt) auf fossilen Rohstoffen, die restlichen Anteile entfielen auf recycelte (9%), biobasierte (1%) und CO₂-basierte Kunststoffe (<1%) (Carus et al., 2020). Gleichzeitig wächst auch der Bedarf an Kunststoffen weltweit. 2019 lag die jährliche CO₂-Emission des gesamten Lebenszyklus von Kunststoffen bei 0,86 Gt, was einer CO₂-Emission von 189 Kohlekraftwerken, die mit voller Leistung (500 MW) laufen, entspricht. Für 2050 wird ein Anstieg auf 2,8 Gt (entspricht 615 Kohlenkraftwerken) prognostiziert (Hamilton et al., 2019).

Einer der Kunststoffe, die auf fossilen Rohstoffen basieren, ist Polyethylenterephthalat (PET), das vor allem in Form von Getränkeverpackungen Anwendung findet. Es handelt sich dabei ein Kondensationspolymer, welches aus Terephthalsäure (1,4-Benzoldicarbonsäure) und Ethylenglycol (Ethan-1,2-diol) unter Wasserabspaltung hergestellt wird.

Terephthalsäure wird großtechnisch durch Oxidation von p-Xylol (1,4-Dimethylbenzol) mit Luftsauerstoff bei ca. 200 °C in Gegenwart von Kobalt-Acetat, Mangan-Acetat und HBr in Essigsäure im sogenannten AMOCO-Prozess hergestellt (Tomäs et al., 2013).

Ethylenglycol wird durch die Hydrolyse von Ethylenoxid (bei 200 °C), welches wiederum durch die Oxidation von Ethen (gewonnen aus fossilen Rohstoffen) erhalten wird, hergestellt (Berger, 2016). Aufgrund der fossilen Quellen der Ausgangsstoffe (p-Xylol und Ethen) kann PET im Allgemeinen nicht als nachhaltig bezeichnet werden.

Um das 1,5-Grad-Ziel des Pariser Klimaabkommens zu erreichen und somit die negativen Folgen des Klimawandels zu begrenzen, müssen nachhaltige Alternativen für die petrochemisch basierten Kunststoffe gefunden werden. Für Ethylenglycol gibt es beispielsweise Verfahren, die auf erneuerbaren Ressourcen aufbauen. So kann beispielsweise Ethen auch durch die Dehydratisierung von Bio-Ethanol, der fermentativ aus Glucose oder Stärke erhalten wurde, hergestellt werden (z.B. Fan et al., 2013). Auch fermentative Umsetzungen von Xylose und/oder Glucose zu Ethylenglycol mittels metabolisch engineerten Mikroorganismen (*Escherichia coli* oder *Saccharomyces cerevisiae*) sind bekannt (Salusjärvi et al., 2019).

Der zweite Baustein von PET, die aromatische Verbindung Terephthalsäure, kann schwer aus nachhaltigen Rohstoffen hergestellt werden und muss daher ersetzt werden. Eine exzellente Substitution für die Terephthalsäure ist die 2,5-Furandicarbonsäure (FDCA), welche überwiegend aus dem katalytischen Upgrading von Biomasse erhalten wird. Durch die Polymerisation von FDCA mit Ethylenglycol entsteht PEF (Polyethylenfuranoat), das in der Struktur anstelle des Benzol-Rings einen heteroaromatischen Furan-Ring trägt. Wie PET ist PEF ein Thermoplast, zeichnet sich aber im Vergleich zu PET durch eine signifikant höhere Bioabbaubarkeit und bessere thermische (höhere Glasübergangstemperatur, niedrigere Schmelztemperatur) sowie mechanische Eigenschaften (höhere Steifheit) aus. Die wichtigste Eigenschaft von PEF aber ist die verringerte Permeabilität für Gase wie O₂ und CO₂. Dies ist besonders für Getränke wichtig, da dadurch die Haltbarkeit von Getränken (Verhinderung von Ausgasen karbonisierter Getränke; Verhinderung von Oxidationsprozessen durch diffundierten Sauerstoff) verlängert werden kann (de Jong et al., 2022).

Ein Überblick über verschiedene chemische Synthesen findet sich im Artikel von Cong et al. (2021).

Der mit Abstand wichtigste Ausgangsstoff für FDCA ist 5-(Hydroxymethyl)furfural (HMF), das beispielsweise aus Cellulose (und damit aus nachwachsenden Rohstoffen) gewonnen werden kann. Durch die enzymatische oder chemische Hydrolyse entsteht aus Cellulose D-Glucose, welche in weiterer Folge zu D-Fructose isomerisiert (enzymatisch oder chemisch) wird. Durch Dehydratisierung (Abspaltung von insgesamt drei H₂O-Molekülen) gelangt man von der D-Fructose zu HMF. Gängige Systeme zur Dehydratisierung von Fructose sind zum einen Mineralsäuren wie H₂SO₄ oder HCl und zum anderen (Brønsted- oder Lewis-)saure Feststoffkatalysatoren (Cong et al., 2021; US 9617234 B1).

HMF weist eine Alkohol- sowie eine Aldehyd-Funktion auf, die zu Carbonsäure-Gruppen oxidiert werden müssen, um FDCA zu erhalten. Die drei dazu notwendigen Oxidationsschritte können auf verschiedene Arten durchgeführt werden: chemisch mit heterogenen bzw. homogenen Katalysatoren, elektrochemisch und biokatalytisch (enzymatisch oder mit ganzen Zellen).

Die direkte Vorstufe von FDCA ist FFA (5-Formyl-2-furancarbonsäure), die beispielsweise durch säurekatalysierte Dehydratisierung aus den Zuckersäure-Derivaten 2-Keto-D-Gluconat (2KGA) oder 5-Keto-D-Gluconat (5KGA) hergestellt werden kann. 2KGA kann durch Behandlung mit HBr in einer Essigsäure/Wasser-Mischung in einem Flussreaktor bei 80 °C zu FFA dehydratisiert werden. In einem abschließenden Schritt kann FFA entweder mit Sauerstoff als Oxidationsmittel sowie mit Metallen (z.B. Pt-Ru/C) oder Metallsalzen (Co- & Mn-Salze) als Katalysatoren oder mit Wasserstoffperoxid zu FDCA oxidiert werden (US 10087161 B2).

Die säurekatalysierte Dehydratisierung von 5KGA zu FFA ist auch in EP 3265450 B1 beschrieben. Für die abschließende Oxidation wird in Essigsäure in Gegenwart von katalytischen Mengen Co-Acetat, Mn-Acetat sowie NaBr mit einem Überdruck von Sauerstoff bei 180 °C (analog zum AMOCO-Prozess) durchgeführt.

Generell zeichnen sich die genannten chemischen Herstellungsverfahren von FDCA ausgehend von FFA als Zwischenstufe durch ungünstige Reaktionsbedingungen (hohe Temperaturen und Drücke), die Bildung von Nebenprodukten sowie die Verwendung von teuren und zum Teil toxischen (Co-Salze) Katalysatoren aus und können daher nicht als nachhaltig bezeichnet werden.

Als Alternative bieten sich biokatalytische Prozesse an, die hochselektiv unter milden Reaktionsbedingungen ablaufen und bioabbaubare Katalysatoren wie Zellen oder Enzyme verwenden (Cong et al., 2021).

FFA kann durch die enzymatische Oxidation von HMF (überwiegend mit O₂ als Oxidationsmittel) hergestellt werden.

Eine Gruppe von Enzymen, die zur Herstellung von FFA aus HMF verwendet werden, sind die Aryl-Alkohol-Oxidasen (AAO, EC 1.1.3.7). Carro et al. (2015) verwendeten eine AAO aus dem Pilz *Pleurotus eryngii,* um 3 mM HMF in 4 h zu FFA (98 mol%) und in geringen Mengen zu FDCA zu oxidieren.

Die durch die AAO katalysierte Oxidation der Aldehyd-Gruppen läuft über die korrespondierenden geminalen Diole (Aldehyd-Hydrate) ab, wobei die Aldehyd-Gruppen in FFA im Vergleich zur Vorstufe 2,5-Diformylfuran (DFF) einen geringeren Hydratationsgrad (DFF: 53%, FFA: 8%) aufweisen, was die präferierte Bildung von FFA anstelle von FDCA erklärt. H₂O₂, das als Nebenprodukt der durch die AAO katalysierten Oxidationen entsteht, oxidiert FFA chemisch zu FDCA (Carro et al., 2015). In einer späteren Studie von Serrano et al. (2019) zur AAO von *P. eryngii* konnte gezeigt werden, dass HMF auch vollständig zu FDCA oxidiert werden kann, wenn eine Katalase zur Entfernung von H₂O₂ eingesetzt wird, da der letzte Oxidationsschritt von FFA zu FDCA durch H₂O₂ inhibiert wird. Auf diese Weise konnten mit einer AAO-Mutante 1,5 mM HMF in 6 d zu 1,6 mM FDCA oxidiert werden.

Weitere AAO sind aus *Mycobacterium* sp. MS1601 (oxidiert 4 g/l (31,7 mM) HMF in 120 h vollständig zu FFA) (Sayed et al., 2022) und aus dem Pilz *Moesziomyces antarcticus* (oxidiert 2 mM HMF in 24 h zu 99,6 mol% FFA und 0,4 mol% FDCA; oxidiert 40 % der FFA (2 mM) in 144 h zu FDCA) (Lappe et al., 2021) bekannt.

Qin et al. (2015) testeten verschiedene Laccasen auf 30 mM HMF mit 20 mol% TEMPO ((2,2,6,6-Tetramethylpiperidin-1-yl)oxyl) als Mediator. Mit der Laccase aus dem Pilz *Panus conchatus* konnten in 96 h 82% des Startmaterials zu FFA oxidiert werden (mit 4% 2,5-Diformylfuran (DFF) und 10% FDCA als Nebenprodukte).

Zhang et al. (2019) immobilisierten eine Laccase (CotA-TJ102) von *Bacillus subtilis* TJ-102 auf magnetischen Nanopartikeln, mit welchen 83,3% des Startmaterials HMF zu FFA (nach 10 Recycling-Zyklen) und einer Selektivität von >96% oxidiert werden konnte.

Jia et al. (2019) nutzten ein Enzymsystem bestehend aus GOase, einer Alkohol-Dehydrogenase aus *Synechocystis* sp. (SADH) sowie HRP, um 97% des Substrats HMF (100 mM) in 48 h zu FFA oxidieren. Neben der Aktivierung der GOase M₃₋₅ dient die HRP auch der Regenerierung von NAD(P)⁺, welches als Kofaktor für die SADH benötigt wird.

US 10344307 B2 legt weitere Enzym(system)e zur Oxidation von HMF zu FFA offen: 1) NAD(P)-abhängige Ketoreduktase & NAD(P)H-Oxidase zur Kofaktor-Regeneration, 2) NAD(P)-abhängige Aldehyd-Dehydrogenase & NAD(P)H-Oxidase sowie 3) Xanthin-Oxidoreduktase (wie die periplasmatische Aldehyd-Oxidoreduktase (PaoABC) aus *E. coli*; oxidiert HMF zu 5-Hydroxymethyl-2-furancarbonsäure (HMFA) sowie DFF zu FFA), Galactose-Oxidase-Variante M₃₋₅ (GOase M₃₋₅; oxidiert HMF zu DFF und HMFA zu FFA) & Meerrettich-Peroxidase (HRP; zur Aktivierung der GOase M₃₋₅). 50 mM DFF können beispielsweise mittels PaoABC in 2 h bei pH 6 vollständig zu FFA oxidiert werden, bei pH 7 und 8 hingegen läuft die Oxidation komplett bis zur FDCA durch. Das unter 3) angeführte Enzymsystem ist auch ausführlich in Zeitschriften-Artikeln (McKenna et al., 2015; McKenna et al., 2017) beschrieben.

Für den abschließenden Oxidationsschritt von FFA zu FDCA sind neben der PaoABC und der AAO auch weitere Enzyme bekannt.

Cajnko et al. (2020) testeten eine Reihe von kommerziell erhältlichen Enzymen (Alkohol-Oxidase (AO) aus *Pichia pastoris*; Galactose-Oxidase aus *Dactylium dendroides*; Katalase aus *Aspergillus niger*; Laccase aus *Trametes versicolor*; eine pilzliche Lignin-Peroxidase (LPO) sowie HRP) auf 10 mM FFA und konnten nur für die AO, für die Laccase sowie für die LPO die Bildung von signifikanten Mengen FDCA (11,6% für AO, 1,1% für Laccase und 3,2% für LPO) nach 72 h beobachten. Als Nebenprodukt wurde 5-Hydroxymethyl-2-furancarbonsäure (HMFA) gefunden (bis zu 18,2% für die AO).

Ein weiteres Enzym zur Oxidation von FFA zu FDCA ist die unspezifische Peroxygenase (UPO, EC 1.11.2.1; benötigt H₂O₂ als Oxidationsmittel) aus *Agrocybe aegerita,* die in Kombination mit der AAO verwendet werden kann, um HMF vollständig zu FDCA zu oxidieren. So können 90% der FFA (3 mM) in 120 h zu FDCA oxidiert werden (Carro et al., 2015). Neben der Oxidation von FFA zu FDCA wird auch die Oxidation von HMF zu FFA über DFF von der UPO katalysiert (Lappe et al., 2021).

Jia et al. (2017) nutzten ein Enzymsystem bestehend aus Pferdeleber-Alkoholdehydrogenase (HLADH) und humanem Hämoglobin (oxidiert mittels H₂O₂ NADH zu NAD⁺), um 96% des Substrats (10 mM FFA) in 60 h zu FDCA oxidieren.

US 8183020 B2 beschreibt die enzymatische Oxidation von FFA zu FDCA mit einer kommerziell erhältlichen Chlorperoxidase aus *Caldariomyces fumago* (EC 1.11.1.10) mit H₂O₂ als Oxidationsmittel.

Aldehyd-Dehydrogenasen (ALDH) sind Enzyme, die die Oxidation von Aldehyd-Gruppen zu Carbonsäure-Gruppen katalysieren. Für den Organismus *Raoultella ornithinolytica* BF60 ist eine ALDH beschrieben, die FFA zu FDCA und HMF zu HMFA oxidieren kann (Hossain et al., 2017).

In US 10344307 B2 ist eine Methode zur Oxidation von DFF mit einer kommerziell erhältlichen ALDH (in Kombination mit einer NAD(P)H-Oxidase) beschrieben, die je nach Bedingung (DFF-Konzentrationen von 10 bis 100 mM) zu FFA, FDCA oder Mischungen führt. Mit steigender DFF-Konzentration wird mehr FFA als FDCA erhalten (10 mM Substrat: 100% FDCA; 50 mM Substrat: 80% FDCA, 20% FFA; 100 mM Substrat: 20% FDCA, 80% FFA (Umsatz jeweils nach 3 h)). Für die Oxidationen werden in US 10344307 B2 zwischen 10 und 50 mol% Kofaktor (bezogen auf die Menge an Substrat) eingesetzt.

Die hier vorgestellten Verfahren weisen im Allgemeinen Nachteile wie niedrige Substratkonzentrationen oder unwirtschaftlich hohe Mengen an zugesetztem Kofaktor auf.

Hier setzt nun die Aufgabe der vorliegenden Erfindung an und setzt sich zum Ziel, ein Verfahren zur Herstellung von FDCA zur Verfügung zu stellen, welches das oben in US 10344307 B2 genannte Verfahren verbessert und insbesondere gestattet, höhere Umsätze bei höheren Substratkonzentrationen bei gleichzeitiger Verringerung der zugesetzten Kofaktor-Mengen zu erzielen.

### Detaillierte Beschreibung der Erfindung

Die Aufgabe der Erfindung wird gelöst, indem 5-Formyl-2-furancarbonsäure (FFA), welche in einer wässrigen Lösung vorliegt, durch Behandeln mit einer NAD(P)⁺-abhängigen Oxidoreduktase *in vitro* zu 2,5-Furandicarbonsäure oxidiert wird, wobei das bei der Oxidation entstandene NAD(P)H enzymatisch mittels einer NAD(P)H-Oxidase wieder zu NAD(P)⁺ oxidiert wird, wonach die Enzyme entfernt werden, dadurch gekennzeichnet, dass eine H₂O-bildende NAD(P)H-Oxidase eingesetzt wird, welche eine Aminosäuresequenz aufweist, die ausgewählt ist aus der Gruppe bestehend aus:
i) einer Aminosäuresequenz, die eine Identität zu SEQ ID Nr. 8 oder SEQ ID Nr. 10 von mindestens 80% aufweist,
ii) einer Aminosäuresequenz, die durch eine Nukleinsäure kodiert wird, die eine Identität zu SEQ ID Nr. 7 oder SEQ ID Nr. 9 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 7 oder SEQ ID Nr. 9 bindet.

FDCA kann im Anschluss durch Kristallisation aus der Lösung abgetrennt werden.

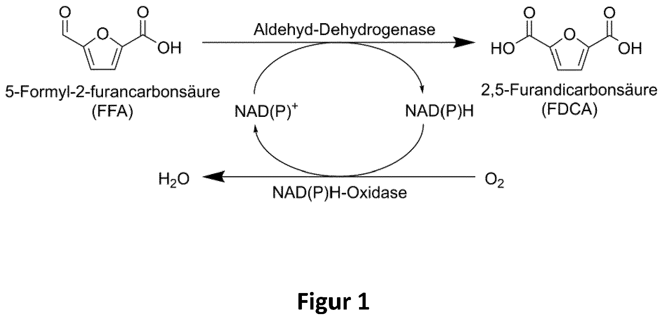

Eine bevorzugte Ausführungsform des erfindungsgemäßen Verfahrens ist in der beiliegenden Figur 1 schematisch dargestellt.

Bei der NAD(P)⁺-abhängigen Oxidoreduktase zur Oxidation von FFA zu FDCA handelt es sich bevorzugt um eine Aldehyd-Dehydrogenase.

Eine NAD(P)⁺-abhängige Oxidoreduktase zur Oxidation von FFA zu FDCA umfasst oder besteht bevorzugt aus einer Aminosäuresequenz, welche ausgewählt ist aus der Gruppe bestehend aus:
i) einer Aminosäuresequenz, die eine Identität zu SEQ ID Nr. 2, SEQ ID Nr. 4 oder SEQ ID Nr. 6 von mindestens 80% aufweist,
ii) einer Aminosäuresequenz, die durch eine Nukleinsäure kodiert wird, die eine Identität zu SEQ ID Nr. 1, SEQ ID Nr. 3 oder SEQ ID Nr. 5 von mindestens 80% aufweist, und iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3 oder SEQ ID Nr. 5 bindet.

SEQ ID Nr. 1: 
ATGTCACGCTATGAACTGCTGATCGATGGTCGCCTGCAGGCGGCCGAGCACTACGACCGGGTGATCGA
CCCGGCCAGCGAAGAAATCGTTGGCGAAGCCGCCCGCGCCAGCCTGGAGCAGGTCGACGCGGCGGTG
GATGCCGCACACCGCGCCTTCCCGGCCTGGGCCACCGATCTCGACGTCCGCCGCCAGAGCCTGGCCCG
AGCCGCCGAACGAGTACGCGAGAATGCCCAGGCGCTGGCCGAGCTGATCACCCGCGAACAGGGTCGC
CCACTGCGCTCGACCCTGGAGGAAGTGGCTGGCGTCGCCGCCACTTTCGAGCACCACGCGCAGCTGGA
GCTACCCGCCGACACCCAGTTGCGCGACGACGGCGAGCGCCTGGTGCGCATCACCCGCAAACCGCTGG
GGGTGGTCGCCGCGATCACCCCGTGGAACGTCCCGCTGATCCTGCTGGTATTGAAGATCGCGCCTGCC
CTGCACGCCGGCAACACCGTGGTGGCCAAGCCCTCGGAGCACACGCCGCTGTCTACCCTGCTGCTGGC
GCGACTGCTGGGCGATGTGTTCCCCGCTGGCGTGTTCAACGTGGTTGCCGGTGCGGGCGAGGTTGGC
GAACACCTGGTACGTCATCCGCGGGTGCGCCACGTGACCTTCACCGGCAGCGTCGCCACCGGCAAGCG
CCTGTATGCCGGCGCGGGGGACGACCTCAAGCGTCTTACCCTGGAACTGGGCGGCAACGACGCCGCG
CTGGTACTGGAAGACGCCGACCTCGACGCCATCGTCGAACCGCTGTTCTGGGGCGCCTTCTGGAACAG
CGGCCAGGTGTGCTTTGCGATCAAGCGCCTGTACGTGCATGACAGCCTGTTCGAACCGTTACTGGCGA
AACTCGCCGAGCGCGCCCAGCGCACCCGCCTCGGTCATGGGCTCGACCCGCAGACGGAACTGGGGCC
GCTGACCAACGCACAGCAACTGGAACGGGTCATCGCCCTGGTGGAAGACGCCAAGGCTCACGGAGCA
CGCATCCGCAGCGGCGGCGTACGGCCCGACGGTCCCGGCTACTTCTACCCGCCGACCCTGGTCAGCGG
CGTGGCGGCCGGCGTCGCGCTGGTGGACGAGGAACAGTTCGGCCCGGTGTTGCCGGTGATCTCCTTCC
 
GCAACGAAGAGGACGCCATTACCCAGGCCAACGCCAGCCACTACGGCCTCGGCGCCTCGGTGTGGACC
CGCGACCTGGCGCGCGGCGAAGCCATCGCCAGGCGGCTGGAGGCGGGCCTGGCCTGGGTCAACCAGC
ACGGCCACATCCAGCCCGGCGCGCCCAAGGGCGGGCACAAGTGGAGCGGGCTCGGCTACGAAGGCG
GGCAGCGCGGCTATGAGGCCTTCAGCGAGCTGCAGGTGCTGAACATTTCGCGGCGCTAA
SEQ ID Nr. 2: 
MSRYELLIDGRLQAAEHYDRVIDPASEEIVGEAARASLEQVDAAVDAAHRAFPAWATDLDVRRQSLARAAE
RVRENAQALAELITREQGRPLRSTLEEVAGVAATFEHHAQLELPADTQLRDDGERLVRITRKPLGVVAAITP
WNVPLILLVLKIAPALHAGNTVVAKPSEHTPLSTLLLARLLGDVFPAGVFNVVAGAGEVGEHLVRHPRVRHV
TFTGSVATGKRLYAGAGDDLKRLTLELGGNDAALVLEDADLDAIVEPLFWGAFWNSGQVCFAIKRLYVHDS
LFEPLLAKLAERAQRTRLGHGLDPQTELGPLTNAQQLERVIALVEDAKAHGARIRSGGVRPDGPGYFYPPTL
VSGVAAGVALVDEEQFGPVLPVISFRNEEDAITQANASHYGLGASVWTRDLARGEAIARRLEAGLAWVNQ
HGHIQPGAPKGGHKWSGLGYEGGQRGYEAFSELQVLNISRR
SEQ ID Nr. 3: 
ATGAAATCGTATCAGGGATTGGCTGACAAGTGGATTAAGGGCAGTGGGGAAGAATACCTTGATATTAA
TCCGGCTGATAAGGATCACGTATTAGCTAAGATAAGATTATATACAAAAGATGACGTTAAAGAAGCTAT
AAACAAGGCTGTAGCCAAATTCGACGAATGGTCAAGGACTCCAGCACCTAAAAGAGGCTCAATATTAC
TTAAGGCAGGGGAATTAATGGAACAAGAAGCCCAAGAGTTTGCGCTATTGATGACATTAGAGGAGGG
TAAGACTCTCAAGGATAGTATGTTTGAAGTGACAAGAAGTTATAATTTACTGAAATTTTATGGAGCATT
AGCATTTAAGATATCTGGGAAAACGCTTCCTTCAGCAGATCCTAATACTAGGATATTTACAGTAAAGGA
ACCCTTAGGCGTAGTAGCTTTAATTACGCCGTGGAATTTCCCATTATCAATACCAGTATGGAAATTGGCT
CCAGCCTTGGCTGCGGGTAACACTGCAGTAATAAAACCAGCGACGAAAACACCGTTAATGGTAGCCAA
ATTGGTAGAAGTGTTGTCTAAAGCTGGATTGCCAGAGGGTGTCGTGAATTTAGTAGTTGGTAAGGGAA
GTGAAGTCGGAGATACCATAGTAAGTGATGATAATATAGCTGCAGTATCATTTACTGGATCAACCGAG
GTAGGTAAGAGAATTTACAAACTCGTAGGAAATAAAAATAGAATGACAAGAATTCAACTAGAGCTAGG
AGGTAAAAACGCGTTATATGTGGATAAGAGCGCTGACTTAACGTTAGCTGCTGAATTAGCCGTAAGAG
GAGGATTTGGACTAACCGGTCAATCATGTACTGCAACTAGTAGGTTAATAATTAACAAGGATGTATATA
CTCAATTTAAACAAAGACTACTAGAAAGAGTTAAGAAGTGGAGAGTAGGACCGGGTACTGAAGATGTT
GATATGGGTCCAGTTGTAGATGAAGGTCAATTTAAGAAAGACTTAGAATATATAGAATACGGAAAGAA
TGTGGGAGCAAAACTAATTTATGGTGGAAATATAATACCAGGGAAGGGATATTTCCTAGAACCTACAA
TTTTCGAAGGAGTCACATCTGATATGAGGCTATTTAAAGAAGAGATTTTCGGTCCAGTACTTAGTGTCA
CTGAGGCAAAAGATTTAGATGAGGCTATAAGGCTAGTTAACGCTGTAGACTATGGACATACAGCTGGA
ATAGTCGCAAGCGATATCAAGGCGATTAACGAGTTCGTTAGTAGGGTAGAGGCAGGAGTTATAAAGG
 
TTAATAAGCCAACAGTCGGACTGGAATTGCAAGCACCATTTGGTGGTTTTAAGAATTCTGGAGCCACTA
CGTGGAAAGAGATGGGAGAAGATGCTTTAGAGTTCTACCTTAAGGAGAAGACAGTATACGAAGGCTG
GTAA
SEQ ID Nr. 4: 
MKSYQGLADKWIKGSGEEYLDINPADKDHVLAKIRLYTKDDVKEAINKAVAKFDEWSRTPAPKRGSILLKAG
ELMEQEAQEFALLMTLEEGKTLKDSMFEVTRSYNLLKFYGALAFKISGKTLPSADPNTRIFTVKEPLGVVALIT
PWNFPLSIPVWKLAPALAAGNTAVIKPATKTPLMVAKLVEVLSKAGLPEGVVNLVVGKGSEVGDTIVSDDNI
AAVSFTGSTEVGKRIYKLVGNKNRMTRIQLELGGKNALYVDKSADLTLAAELAVRGGFGLTGQSCTATSRLII
NKDVYTQFKQRLLERVKKWRVGPGTEDVDMGPVVDEGQFKKDLEYIEYGKNVGAKLIYGGNIIPGKGYFLE
PTIFEGVTSDMRLFKEEIFGPVLSVTEAKDLDEAIRLVNAVDYGHTAGIVASDIKAINEFVSRVEAGVIKVNKPT
VGLELQAPFGGFKNSGATTWKEMGEDALEFYLKEKTVYEGW
SEQ ID Nr. 5: 
ATGTCGACATTTCATTTACTTATTGATGGCCATCTGCAAGCCAGTGACCAATCCGATGTGGTGATCAACC
CCGCGACCGAGCTGGAAGTCGGGCGTGCCCCACGTGCCAGCGCCACCCAGGTCGACCAGGCAGTGGA
AGCCGCACATCAGGCATTTCATCGCTGGGCATCACAGCCTGAGGTGCGCCAGCAGGCACTGCTGGGCG
CGGCCGCCGCCATTCGCCAGCATGCCGATGCCCTGGCACGCCTGATCACGCAAGAGCAGGGGCGGCCA
TTGCACTTTACCCAGGGCGAGGTGGCCGGGGCTGCTGCTACCTTTGAACACTATGCCGGGTTTGCCGCG
CCATCGGATGTGGTGCTGCAGCAGGACGAACAAAAGCGGGTCAGCATTGAGCGCAGGCCGTTTGGCG
TAGTGGCTGCCATTACGCCCTGGAATGTGCCCATCATCCTGCTGGTACTGAAAATCGCCCCGGCCTTGA
AGGCTGGCAATACCGTGGTCGCCAAGCCATCGGAATACACCCCGCTTTCTACCTTGTACCTGGGTGAAA
TTCTGAAAGATGTATTTCCGCCCGGCGTGCTGAACGTGATAGCCGGTGACGGTCAGGTGGGGGCGCGC
CTGGCATCGCATCCGCTGGTGCAGAAGGTGACGTTCACCGGCAGCGTGGCAACCGGAAAAAAACTCTA
TGCCAGCGCCGCGCAGGATGTAAAACGCCTGACGCTGGAACTGGGCGGTAACGATGCGGCCATCGTG
CTGGACGATGCCAATGTCGATGCCATTGCCGAGAAGATTTTCTGGGGCGCCTTCTGGAATAGCGGTCA
GGTGTGCTTTGCCATCAAGCGCCTCTATGTGCACGAGCGTGTTTTCCAGCCCTTGCTCGATGCCCTGGT
GAAACGCGCGCAAAAAACCCGCGTGGGCGATGGCCAGCTGCCGGGTACCGAGCTGGGGCCGCTTACC
AACAAAGCCCAGTTTGAGCGCGTGATATCGCTGGTGGAAGACGCCAGACGCCATGGCGCCACCATTCA
TTCAGGCGGTGCTGCATTGCCTGGCCCCGGCTATTTCTATCCGCCCACCCTGGTCACCGGCATAGGCGC
AGGTGTCGCGCTGGTGGATGAAGAGCAGTTCGGACCAGTATTGCCGCTGATTCCTTTCCGTGATGAGC
AAGAGGCGGTGCGTCAGGCCAACGACAGTCCGTTTGGTCTGGGCGCTTCGGTTTGGACCGCCAATCCC
GAACGCGGCCTGGCGCTGGTACGCCAGCTCCAGGCCGGGCTCGCCTGGGTCAACCAGCATGGCGATAT
 
CCATCCCGGTGCGCCCAAGGGCGGCTACAAATCCAGTGGCCTTGGCTACGAGGGCGGGCTGCGTGGCT
ATGACGAGTTCAGCGAGCTGCAAGTCGTCAATGCGGCGCTGGTTTAA
SEQ ID Nr. 6:

MSTFHLLIDGHLQASDQSDVVINPATELEVGRAPRASATQVDQAVEAAHQAFHRWASQPEVRQQALLGA
AAAIRQHADALARLITQEQGRPLHFTQGEVAGAAATFEHYAGFAAPSDVVLQQDEQKRVSIERRPFGVVAA
ITPWNVPIILLVLKIAPALKAGNTVVAKPSEYTPLSTLYLGEILKDVFPPGVLNVIAGDGQVGARLASHPLVQK
VTFTGSVATGKKLYASAAQDVKRLTLELGGNDAAIVLDDANVDAIAEKIFWGAFWNSGQVCFAIKRLYVHE
RVFQPLLDALVKRAQKTRVGDGQLPGTELGPLTNKAQFERVISLVEDARRHGATIHSGGAALPGPGYFYPPT
LVTGIGAGVALVDEEQFGPVLPLIPFRDEQEAVRQANDSPFGLGASVWTANPERGLALVRQLQAGLAWVN
QHGDIHPGAPKGGYKSSGLGYEGGLRGYDEFSELQVVNAALV

Die hier angeführten Oxidoreduktasen zur Oxidation von FFA zu FDCA umfassen vorzugsweise eine Aminosäuresequenz, die eine Identität zu SEQ ID Nr. 2, SEQ ID Nr. 4 oder SEQ ID Nr. 6 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist.

Alternativ umfassen die Oxidoreduktasen zur Oxidation von FFA zu FDCA vorzugsweise eine Aminosäuresequenz, die durch eine Nukleinsäure kodiert wird, die eine Identität zu SEQ ID Nr. 1, SEQ ID Nr. 3 oder SEQ ID Nr. 5 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäßen Oxidoreduktase zur Oxidation von FFA zu FDCA kodiert, die Nukleinsäuresequenz SEQ ID Nr. 1, SEQ ID Nr. 3 oder SEQ ID Nr. 5 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990) verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 11, eine Erwartung (E) von 10, M = 5, N = -4 und einen Vergleich beider Stränge. Für Aminosäuresequenzen verwendet das blastp-Programm als Vorgaben eine Wortlänge von 3 und eine Erwartung (E) von 10 und die BLOSUM62-Scoring-Matrix (Henikoff & Henikoff, 1989), Alignments (B) von 50, Erwartung (E) von 10, M = 5, N = -4.

Alternativ umfassen die Oxidoreduktasen zur Oxidation von FFA zu FDCA vorzugsweise eine Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3 oder SEQ ID Nr. 5 bindet. Wie hierin verwendet, beziehen sich die stringenten Bedingungen auf Bedingungen, unter denen sogenannte spezifische Hybride, jedoch keine unspezifischen Hybride gebildet werden. Beispielsweise umfassen die stringenten Bedingungen eine Hybridisierung in 6xSSC (Natriumchlorid/Natriumcitrat) bei 45 °C und dann Waschen mit 0,2 bis 1xSSC, 0,1% SDS bei 50 bis 65 °C; oder derartige Bedingungen können eine Hybridisierung in 1xSSC bei 65 bis 70 °C und dann Waschen mit 0,3xSSC bei 65 bis 70 °C umfassen. Die Hybridisierung kann durch herkömmlich bekannte Verfahren, wie etwa jene, die von J. Sambrook et al. in Molecular Cloning, A Laboratory Manual, 2nd Ed., Cold Spring Harbor Laboratory (1989), beschrieben sind, durchgeführt werden.

Offenbart ist die Verwendung einer Oxidoreduktase zur Oxidation von FFA zu FDCA, wobei die Oxidoreduktase eine Aminosäuresequenz umfasst, welche ausgewählt ist aus der Gruppe bestehend aus:
i) einer Aminosäuresequenz, die eine Identität zu SEQ ID Nr. 2, SEQ ID Nr. 4 oder SEQ ID Nr. 6 von mindestens 80% aufweist,
ii) einer Aminosäuresequenz, die durch eine Nukleinsäure kodiert wird, die eine Identität zu SEQ ID Nr. 1, SEQ ID Nr. 3 oder SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3 oder SEQ ID Nr. 5 bindet.

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Eine besonders bevorzugt eingesetzte H₂O-bildende NAD(P)H-Oxidase umfasst oder besteht vorzugsweise aus eine Aminosäuresequenz, die ausgewählt ist aus der Gruppe bestehend aus:
i) einer Aminosäuresequenz, die eine Identität zu SEQ ID Nr. 8 oder SEQ ID Nr. 10 von mindestens 80% aufweist,
ii) einer Aminosäuresequenz, die durch eine Nukleinsäure kodiert wird, die eine Identität zu SEQ ID Nr. 7 oder SEQ ID Nr. 9 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 7 oder SEQ ID Nr. 9 bindet.

SEQ ID Nr. 7: 
ATGAAAGTAGTAGTAGTAGGCTGTACACATGCAGGAACAGCGGCAGTTAAGACGATTTTAAATGAACA
TCCAGATGCATCAGTATCAGTATATGAGCGTAATGACAATGTCTCATTTCTATCTTGTGGGATTGCGTTG
TATGTTGGTGGAGTTGTGAAAGATCCTGCAGGTTTGTTTTATTCAAGTCCAGAAGAACTTGCATCAATG
GGCGCGAAAATTAACATGGAACACAATGTGAAAAATATAGATAATGAGAATAAGGTCGTAGTAATTGA
GAATTTAAAAACAGGCGAAACATTTGAAGAAAGCTATGATAAGTTGGTAATGACAACTGGATCATGGC
CAATTATTCCTCCAATTGATGGAATCAATAGTGAAAATATTCTTTTGTGTAAAAACTATAACCAAGCAAA
TGAAATTATTAAAGAATCAAAAAATGCTAAAAAGATTGTCATTGTTGGTGGTGGCTATATTGGAATTGA
ATTAGTTGAGGCATTTGCAGAATCTGGCAAGCAAGTGACGCTAGTTGATGGATTAGATCGTATTTTAAA
CAAATATTTAGATGCTGAATTCACTTCTGTTTTAGAGCATGATTTACAAGAAAGAGGCGTTACGCTAGCT
TTAAACCAAACCGTCGAGAAATTTGTTGCCAATGAATCAGGTGCTGTGACAGCTGTGAAAACACCAGTT
GGAGAATATGAGGCTGATTTAGTTATTTTATGTGTTGGATTTAAACCAAATACTGATTTGTTGAAGGAT
AAAGTAGAGATGTTGCCAAATGGTGCCATCGTAGTGGATGAATATATGAGAACAAGCGATGAAGCGAT
TTTTGCTGCTGGCGATAGTTGCGCGGTTCATTATAATCCAACTGGAGGCTCTGCGTATATTCCGTTAGCT
ACAAATGCAGTTAGAATGGGAGCTTTAGTTGGGAAAAATATTGTTTCTCCAACAGTTAAATATCGTGGC
ACGCAAGCAACTTCTGGTTTATATTTATTTGGTTTTAATATAGGTTCAACCGGATTGACTGAAAATAGCG
CTCCTCATTTTGGCGTAGAGGTTCGTTCAGTAGTTGTAGAAGATAATTATCGTCCAGAGTTTATGCCGAC
AACAGAGAAAGTAACGATGAAATTAGTTTATGAAGTAGGAACGAATCGGATTGTTGGAGGTCAAATCA
TGTCAAAATATGATGTGACACAATCTGCCAATACGTTATCTTTATGTGTTCAAAATAAAATGACGATTGA
GGATTTGGCTTATGTAGATTTCTTCTTCCAACCTCACTTTGATCGTCCTTGGAACTATTTAAATATTTTAG
CGCAAGCAGCTGTTGAGCAAGAGCGTAAACTAGCAAAATAA
SEQ ID Nr. 8: 
MKVVVVGCTHAGTAAVKTILNEHPDASVSVYERNDNVSFLSCGIALYVGGVVKDPAGLFYSSPEELASMGA
KINMEHNVKNIDNENKVVVIENLKTGETFEESYDKLVMTTGSWPIIPPIDGINSENILLCKNYNQANEIIKESK
NAKKIVIVGGGYIGIELVEAFAESGKQVTLVDGLDRILNKYLDAEFTSVLEHDLQERGVTLALNQTVEKFVANE
SGAVTAVKTPVGEYEADLVILCVGFKPNTDLLKDKVEMLPNGAIVVDEYMRTSDEAIFAAGDSCAVHYNPT
GGSAYIPLATNAVRMGALVGKNIVSPTVKYRGTQATSGLYLFGFNIGSTGLTENSAPHFGVEVRSVVVEDNY
RPEFMPTTEKVTMKLVYEVGTNRIVGGQIMSKYDVTQSANTLSLCVQNKMTIEDLAYVDFFFQPHFDRPW
NYLNILAQAAVEQERKLAK
SEQ ID Nr. 9: 
ATGAGCAAAATTGTTATCGTGGGTGCAAATCATGCAGGCACCGCAGCAATTAATACCATTCTGGATAAT
TATGGCAGCGAAAATGAAGTGGTTGTGTTTGATCAGAATAGCAACATTAGCTTTCTGGGTTGTGGTATG
GCACTGTGGATTGGTAAACAAATTAGCGGTCCGCAGGGTCTGTTTTATGCAGATAAAGAAAGCCTGGA
AGCAAAAGGTGCCAAAATCTATATGGAAAGTCCGGTTACCGCCATTGATTATGATGCAAAACGTGTTAC
CGCACTGGTTAATGGTCAAGAACATGTTGAAAGCTACGAAAACTGATTCTGGCAACCGGTAGCACCC
CGATTCTGCCTCCGATTAAAGGTGCAGCCATTAAAGAAGGTAGTCGCGATTTTGAAGCAACCCTGAAAA
ATCTGCAGTTCGTGAAACTGTATCAGAATGCCGAAGATGTGATTAACAAACTGCAGGATAAAAGCCAG
AATCTGAATCGTATTGCAGTTGTTGGTGCAGGTTATATTGGTGTTGAACTGGCAGAAGCATTTAAACGT
CTGGGTAAAGAAGTGATTCTGATTGACGTTGTTGATACCTGTCTGGCAGGTTATTATGATCAGGATCTG
AGCGAAATGATGCGTCAGAATCTGGAAGATCATGGTATCGAACTGGCATTTGGTGAAACCGTTAAAGC
AATTGAAGGTGATGGTAAAGTGGAACGTATTGTTACCGATAAAGCAAGCCATGATGTGGATATGGTTA
TTCTGGCAGTTGGTTTTCGTCCGAATACAGCACTGGGTAATGCAAAACTGAAAACCTTTCGTAATGGTG
CCTTTCTGGTGGATAAAAAACAAGAAACCAGCATCCCGGATGTTTATGCAATTGGTGATTGTGCAACCG
TGTATGATAATGCCATTAACGACACCAACTATATTGCACTGGCAAGCAATGCACTGCGTAGCGGTATTG
TTGCAGGTCATAATGCAGCCGGTCATAAACTGGAAAGTCTGGGTGTTCAGGGTAGCAATGGTATTTCA
ATTTTTGGCCTGAATATGGTTAGCACCGGTCTGACCCAAGAAAAAGCCAAACGTTTTGGTTATAATCCG
GAAGTTACCGCCTTTACCGATTTTCAGAAAGCCAGCTTTATCGAGCATGATAACTATCCGGTTACGCTGA
AAATTGTGTATGACAAAGATAGCCGTCTGGTTCTGGGTGCACAGATGGCCAGCAAAGAAGATATGAGC
ATGGGTATTCACATGTTTAGCCTGGCCATTCAAGAGAAAGTTACCATTGAACGTCTGGCCCTGCTGGAT
TATTTCTTTCTGCCGCATTTTAATCAGCCGTACAACTATATGACCAAAGCAGCACTGAAAGCCAAATAA
SEQ ID Nr. 10:

MSKIVIVGANHAGTAAINTILDNYGSENEVVVFDQNSNISFLGCGMALWIGKQISGPQGLFYADKESLEAKG
AKIYMESPVTAIDYDAKRVTALVNGQEHVESYEKLILATGSTPILPPIKGAAIKEGSRDFEATLKNLQFVKLYQN

AEDVINKLQDKSQNLNRIAVVGAGYIGVELAEAFKRLGKEVILIDVVDTCLAGYYDQDLSEMMRQNLEDHGI
ELAFGETVKAIEGDGKVERIVTDKASHDVDMVILAVGFRPNTALGNAKLKTFRNGAFLVDKKQETSIPDVYAI
GDCATVYDNAINDTNYIALASNALRSGIVAGHNAAGHKLESLGVQGSNGISIFGLNMVSTGLTQEKAKRFGY
NPEVTAFTDFQKASFIEHDNYPVTLKIVYDKDSRLVLGAQMASKEDMSMGIHMFSLAIQEKVTIERLALLDYF
FLPHFNQPYNYMTKAALKAK

Die bevorzugt verwendete H₂O-bildende NAD(P)H-Oxidase umfasst oder besteht vorzugsweise aus einer Aminosäuresequenz, die eine Identität zu SEQ ID Nr. 10 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die H₂O-bildende NAD(P)H-Oxidase die Aminosäuresequenz SEQ ID Nr. 8 oder SEQ ID Nr. 10 oder besteht aus dieser.

Alternativ umfasst die H₂O-bildende NAD(P)H-Oxidase vorzugsweise eine Aminosäuresequenz, die durch eine Nukleinsäure kodiert wird, die eine Identität zu SEQ ID Nr. 7 oder SEQ ID Nr. 9 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die H₂O-bildende NAD(P)H-Oxidase kodiert, die Nukleinsäuresequenz SEQ ID Nr. 7 oder SEQ ID Nr. 9 oder besteht aus dieser.

Ein weiterer Aspekt der vorliegenden Erfindung betrifft die Verwendung einer H₂O-bildenden NAD(P)H-Oxidase, welche eine Aminosäuresequenz umfasst oder daraus besteht, die ausgewählt ist aus der Gruppe bestehend aus:
i) einer Aminosäuresequenz, die eine Identität zu SEQ ID Nr. 8 oder SEQ ID Nr. 10 von mindestens 80% aufweist,
ii) einer Aminosäuresequenz, die durch eine Nukleinsäure kodiert wird, die eine Identität zu SEQ ID Nr. 7 oder SEQ ID Nr. 9 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 7 oder SEQ ID Nr. 9 bindet.

In weiteren bevorzugten Ausführungsform des erfindungsgemäßen Verfahrens beträgt die Konzentration von FFA in der wässerigen Lösung 5 - 100 g/l.

Der besonders bevorzugte Temperaturbereich liegt zwischen 15 und 40 °C.

Der besonders bevorzugte pH-Bereich der Reaktion liegt zwischen pH 5 und pH 9.

Bei einer weiteren, bevorzugten Variante des erfindungsgemäßen Verfahrens liegen die Enzyme in einer Suspension, im Homogenat und/oder im Lysat der entsprechenden, sie bildenden Zellen vor, wobei Lysate besonders bevorzugt sind.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund der Entfernung von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme* & *Herstellung der Lysate* für Details).

In einer weiteren Variante können die Enzyme auch mit einem wasserlöslichen Polymer wie Polyethylenglycol am N-Terminus modifiziert, in oder auf einer festen Matrix immobilisiert oder Teil eines Fusionsproteins sein.

In einer weiteren Variante können die Enzyme in Pulverform, in lyophilisierter oder sprühgetrockneter Form vorliegen.

Mit dem nachfolgenden Beispiel wird die bevorzugte Ausführungsformen der Erfindung noch näher beschrieben.

### Materialien

5-Formyl-2-furancarbonsäure (FFA) sowie 2,5-Furandicarbonsäure (FDCA) wurden von TCI, Kaliumdihydrogenphosphat, di-Kaliumhydrogenphosphat sowie Natriumdodecylsulfat (SDS) wurden von Carl Roth, NAD⁺, NADH-Dinatriumsalz, NADP⁺-Dinatriumsalz, NADPH-Tetranatriumsalz sowie Acetonitril wurden von PanReac AppliChem (ITW Reagents) und Triethanolamin wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia coli*-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente E. coli-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (z.B. Triethanolamin (TEA) - HCl) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Enzymtypen und Spenderorganismen für die in den Beispielen verwendeten Enzyme (ALDH = Aldehyd-Dehydrogenase).**

| **Enzymtyp (EC-Klasse)** | **katalysierte Reaktion** | **Spenderorganismus** | **Literatur** |
|---|---|---|---|
| ALDH I | FFA → FDCA | *Pseudomonas nitroreducens* | (NCBI Protein Database: WP_024766379.1); SEQ ID Nr. 2 |
| ALDH II * | FFA → FDCA | *Saccharolobus solfataricus* | (NCBI Protein Database: WP_009990943.1); SEQ ID Nr. 4 |
| ALDH III | FFA → FDCA | *Methylovorus glucosotrophus* | (NCBI Protein Database: WP_015829138.1); SEQ ID Nr. 6 |
| NADH-Oxidase I (EC 1.6.3.4) | NADH → NAD⁺ | *Carnobacterium divergens* | SEQ ID Nr. 8 |
| NADH-Oxidase II (EC 1.6.3.4) | NADH → NAD⁺ | *Streptococcus mutans* | (Matsumoto et al., 1996); SEQ ID Nr. 10 |

| | | | |
|---|---|---|---|
| *Diese ALDH wird in der NCBI Protein Database (Eintrag WP_009990943.1) als 2,5-Dioxopentanoat-Dehydrogenase (katalysiert die Oxidation von 2,5-Dioxopentanoat zu α-Ketoglutarat) klassifiziert. | | | |

### Analytische Methoden

### High Performance Liquid Chromatography (HPLC)

Zur Quantifizierung von FFA und FDCA wurde HPLC (High Performance Liquid Chromatography) verwendet. Detektion erfolgt mittels eines UV-Detektors. Zur Messung wird eine Phenomenex Rezex ROA-Organic Acid H+ (8%) Säule mit entsprechender Vorsäule verwendet und mit 1 mM Schwefelsäure isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung oder der Verbrauch von NAD(P)H bei einer Wellenlänge von 340 nm über die Änderung der Absorption verfolgt. Die Messungen wurden mit 0,2 mM Kofaktor (NAD(P)⁺ oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH/NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von 5-Formyl-2-furancarbonsäure zu 2,5-Furandicarbonsäure mit der Aldehyd-Dehydrogenase I (ALDH I)

Die Reaktion wurde in einem BioXplorer Tisch-Bioreaktor mit Polyblock (H.E.L.) durchgeführt. Als Gefäß wurde ein Edelstahlreaktor (max. Volumen 400 ml) mit aufgesetztem Rührwerk und pH-Elektrode verwendet. Die pH-Kontrolle erfolgte durch die Zudosierung von 5M NaOH oder 1M H₂SO₄.

Zu Beginn wurden 5,5 g FFA in 150 ml eines 100 mM Kalium-Phosphat-Puffers (pH 7) vorgelegt und unter Rühren auf 20 °C gebracht. Danach wurden 20 ml ALDH I-Lysat, 13 ml NADH-Oxidase-Lysat sowie 3 ml einer 10 mM NAD⁺-Lösung (finale Konzentration 0,2 mM) eingebracht. Zusätzlich wurde ein Überdruck von 320 mbar (O₂-Atmosphäre) angelegt.

Zur Analyse wurden 50 µl des Ansatzes mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 250 µl des Überstands wurden in einem HPLC-Vial mit 750 µl einer Acetonitril/Wasser-Mischung (1/4 v/v) verdünnt und mittels HPLC (UV-Detektion) vermessen.

Nach 20 h wurde die FFA vollständig zu FDCA oxidiert.

Zur Aufarbeitung wurde der Reaktorinhalt auf 70 °C aufgeheizt und für 1 h bei dieser Temperatur gerührt. Nach dem Abzentrifugieren von denaturiertem Protein, wurde der Überstand durch einen Faltenfilter filtriert. Dadurch wurde eine klare Lösung erhalten, die durch Ansäuern mit 10 ml einer 12 M H₂SO₄-Lösung auf pH < 2 gebracht wurde. Durch Abkühlen auf 4 °C bildete sich ein Niederschlag aus FDCA, welcher abfiltriert wurde und bei 50 °C im Vakuumtrockenschrank getrocknet wurde.

### Beispiel 2

### Oxidation von 5-Formyl-2-furancarbonsäure zu 2,5-Furandicarbonsäure mit der Aldehyd-Dehydrogenase II (ALDH II)

In einem Glas-Vial wurden folgende Komponenten vermischt: 300 µl einer FFA-Lösung (11,9 g/l), 60 µl deionisiertes Wasser, 50 µl ALDH II-Lysat, 50 µl eines 1 M Kaliumphosphat-Puffers (pH 8) sowie 40 µl NADH-Oxidase-Lysat. Der Ansatz wurde unter kontinuierlichem Schütteln (Eppendorf-Thermomixer; 20 °C, 800 rpm) für insgesamt 20 h inkubiert.

Zur Analyse wurden 50 µl des Ansatzes mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (UV-Detektion) vermessen.

Auf diese Weise wurden 34,5% der FFA (7,1 g/l) zu FDCA (gefundene Konzentration: 2,4 g/l) oxidiert.

### Beispiel 3

### Oxidation von 5-Formyl-2-furancarbonsäure zu 2,5-Furandicarbonsäure mit der Aldehyd-Dehydrogenase III (ALDH III)

In einem Glas-Vial wurden folgende Komponenten vermischt: 5,1 mg FFA, 325 µl deionisiertes Wasser, 35 µl ALDH III-Lysat, 100 µl eines 500 mM Kaliumphosphat-Puffers (pH 8), 30 µl NADH-Oxidase-Lysat, sowie 5 µl einer 10 mM NAD⁺-Lösung. Der Ansatz wurde unter kontinuierlichem Schütteln (Eppendorf-Thermomixer; 20 °C, 800 rpm) für insgesamt 24 h inkubiert.

Zur Analyse wurden 50 µl des Ansatzes mit 200 µl Acetonitril versetzt und im Eppendorf Thermomixer bei 85 °C und 1200 rpm für 15 min inkubiert. Die Probe wurde in einer Zentrifuge kurz zentrifugiert, mit 750 µl deionisiertem Wasser versetzt, gevortext und anschließend für 5 min bei max. g zentrifugiert. 200 µl des Überstands wurden in ein HPLC-Vial mit Insert überführt und mittels HPLC (UV-Detektion) vermessen.

Auf diese Weise wurden 57,0% der FFA (10,2 g/l) zu FDCA (gefundene Konzentration: 5,9 g/l) oxidiert.

### Literatur

Carus, M., Dammer, L., Raschka, A., Skoczinski, P., & vom Berg, C. (2020). Renewable Carbon-Key to a Sustainable and Future-Oriented Chemical and Plastic Industry. https://renewable-carbon-initiative.com/wp-content/uploads/2020/09/20-09-21_Paper_12-on-Renewable-Carbon.pdf (Zugriff am 17.10.2022)
Hamilton, L. A., Feit, S., Muffett, C., Kelso, M., Rubright, S. M., Bernhardt, C., Schaeffer, E., Moon, D., Morris, J., & Labbé-Bellas, R. (2019). Plastic & Climate: The Hidden Costs of a Plastic Planet. https://www.ciel.org/wp-content/uploads/2019/05/Plastic-and-Climate-FINAL-2019.pdf (Zugriff am 18.10.2022)
Tomäs, R. A. F., Bordado, J. C. M., & Gomes, J. F. P. (2013). p-Xylene Oxidation to Terephthalic Acid: A Literature Review Oriented toward Process Optimization and Development. Chemical Reviews, 113(10), 7421-7469. https://doi.org/10.1021/cr300298j
Berger, A. (2016). Ethylenglycol, RD-05-01999 in Böckler, F., Dill, B., Eisenbrand, G., Faupel, F., Fugmann, B., Gamse, T., Matissek, R., Pohnert, G., Rühling, A., Schmidt, S., Sprenger, G. (Ed.), RÖMPP [Online], Stuttgart, Georg Thieme Verlag. https://roempp.thieme.de/lexicon/RD-05-01999 (aufgerufen am 22.12.2023)
Fan, D., Dai, D.-J., Wu, H.-S. (2013). Ethylene Formation by Catalytic Dehydration of Ethanol with Industrial Considerations. Materials 6(1), 101-115. https://doi.org/10.3390/ma6010101
Salusjärvi, L., Havukainen, S., Koivistoinen, O., & Toivari, M. (2019). Biotechnological production of glycolic acid and ethylene glycol: current state and perspectives. Applied Microbiology and Biotechnology, 103, 2525-2535. https://doi.org/10.1007/s00253-019-09640-2
de Jong, E., Visser, H. A., Dias, A. S., Harvey, C., Gruter, G.-J. M. (2022). The Road to Bring FDCA and PEF to the Market. Polymers, 14, 943. https://doi.org/10.3390/polym14050943
Cong, H., Yuan, H., Tao, Z., Bao, H., Zhang, Z., Jiang, Y., Huang, D., Liu, H., & Wang, T. (2021). Recent Advances in Catalytic Conversion of Biomass to 2,5-Furandicarboxylic Acid. Catalysts, 11(9), 1113. https://doi.org/10.3390/catal11091113
Carro, J., Ferreira, P., Rodriguez, L., Prieto, A., Serrano, A., Balcells, B., Ardá, A., Jiménez-Barbero, J., Gutiérrez, A., Ullrich, R., Hofrichter, M., & Martinez, A. T. (2015). 5-hydroxymethylfurfural conversion by fungal aryl-alcohol oxidase and unspecific peroxygenase. FEBS Journal, 282(16), 3218-3229. https://doi.org/10.1111/febs.13177
Serrano, A., Calviño, E., Carro, J., Sänchez-Ruiz, M. I., Cañada, F. J., & Martinez, A. T. (2019). Complete oxidation of hydroxymethylfurfural to furandicarboxylic acid by aryl-alcohol oxidase. Biotechnology for Biofuels and Bioproducts, 12, 217. https://doi.org/10.1186/s13068-019-1555-z
Sayed, M., Gaber, Y., Junghus, F., Martin, E. V., Pyo, S.-H., & Hatti-Kaul, R. (2022). Oxidation of 5-hydroxymethylfurfural with a novel aryl alcohol oxidase from Mycobacterium sp. MS1601. Microbial Biotechnology, 15(8), 2176-2190. https://doi.org/10.1111/1751-7915.14052
Lappe, A., Jankowski, N., Albrecht, A., Koschorreck, K. (2021). Characterization of a thermotolerant aryl-alcohol oxidase from Moesziomyces antarcticus oxidizing 5-hydroxymethyl-2-furancarboxylic acid. Applied Microbiology and Biotechnology, 105, 8313-8327. https://doi.org/10.1007/s00253-021-11557-8
Qin, Y.-Z., Li, Y.-M., Zong, M.-H., Wu, H., & Li, N. (2015). Enzyme-catalyzed selective oxidation of 5-hydroxymethylfurfural (HMF) and separation of HMF and 2,5-diformylfuran using deep eutectic solvents. Green Chemistry, 17(7), 3718-3722. https://doi.org/10.1039/C5GC00788G
Zhang, C., Chang, X., Zhu, L., Xing, Q., You, S., Qi, W., Su, R., & He, Z. (2019). Highly efficient and selective production of FFCA from CotA-TJ102 laccase-catalyzed oxidation of 5-HMF. International Journal of Biological Macromolecules, 128, 132-139. https://doi.org/10.1016/j.ijbiomac.2019.01.104
Jia, H.-Y., Zong, M.-H., Zheng, G.-W., & Li, N. (2019). One-Pot Enzyme Cascade for Controlled Synthesis of Furancarboxylic Acids from 5-Hydroxymethylfurfural by H2O2 Internal Recycling. ChemSusChem, 12(21), 4764-4768. https://doi.org/10.1002/cssc.201902199
McKenna, S. M., Leimkühler, S., Herter, S., Turner, N. J., & Carnell, A. J. (2015). Enzyme cascade reactions: Synthesis of furandicarboxylic acid (FDCA) and carboxylic acids using oxidases in tandem. Green Chemistry, 17, 3271-3275. https://doi.org/10.1039/C5GC00707K
McKenna, S. M., Mines, P., Law, P., Kovacs-Schreiner, K., Birmingham, W. R., Turner, N. J., Leimkühler, S., & Carnell, A. J. (2017). The continuous oxidation of HMF to FDCA and the immobilisation and stabilisation of periplasmic aldehyde oxidase (PaoABC). Green Chemistry, 19, 4660-4665. https://doi.org/10.1039/C7GC01696D
Cajnko, M. M., Novak, U., Grilc, M., & Likozar, B. (2020). Enzymatic conversion reactions of 5-hydroxymethylfurfural (HMF) to bio-based 2,5-diformylfuran (DFF) and 2,5-furandicarboxylic acid (FDCA) with air: mechanisms, pathways and synthesis selectivity. Biotechnology for Biofuels, 13, 66. https://doi.org/10.1186/s13068-020-01705-z
Jia, H.-Y., Zong, M.-H., Yu, H.-L., & Li, N. (2017). Dehydrogenase-Catalyzed Oxidation of Furanics: Exploitation of Hemoglobin Catalytic Promiscuity. ChemSusChem, 10(18), 3524-3528. https://doi.org/10.1002/cssc.201701288
Hossain, G. S., Yuan, H., Li, J., Shin, H., Wang, M., Du, G., Chen, J., & Liu, L. (2017). Metabolic Engineering of Raoultella ornithinolytica BF60 for Production of 2,5-Furandicarboxylic Acid from 5-Hydroxymethylfurfural. Applied and Environmental Microbiology, 83(1), e02312-16. https://doi.org/10.1128/AEM.02312-16
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Lin, B., & Tao, Y. (2017). Whole-cell biocatalysts by design. Microbial Cell Factories, 16, 106. https://doi.org/10.1186/s12934-017-0724-7
Protein [Internet]. Bethesda (MD): National Library of Medicine (US), National Center for Biotechnology Information. Accession No. WP_024766379.1, aldehyde dehydrogenase family protein [Pseudomonas nitroreducens]. Verfügbar unter: https://www.ncbi.nlm.nih.gov/protein/WP_024766379.1/ (Zugriff am 21.12.2023)
Protein [Internet]. Bethesda (MD): National Library of Medicine (US), National Center for Biotechnology Information. Accession No. WP_009990943.1, 2,5-dioxopentanoate dehydrogenase [Saccharolobus solfataricus]. Verfügbar unter: https://www.ncbi.nlm.nih.gov/protein/WP_009990943.1/ (Zugriff am 21.12.2023)
Protein [Internet]. Bethesda (MD): National Library of Medicine (US), National Center for Biotechnology Information. Accession No. WP_015829138.1, aldehyde dehydrogenase family protein [Methylovorus glucosotrophus]. Verfügbar unter: https://www.ncbi.nlm.nih.gov/protein/WP_015829138.1/ (Zugriff am 21.12.2023)
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39

## Patentansprüche

1. Verfahren zur Herstellung von 2,5-Furandicarbonsäure, indem 5-Formyl-2-furancarbonsäure, welche in einer wässrigen Lösung vorliegt, durch Behandeln mit einer NAD(P)⁺-abhängigen Oxidoreduktase *in vitro* zu 2,5-Furandicarbonsäure oxidiert wird, wobei das bei der Oxidation entstandene NAD(P)H enzymatisch mittels einer NAD(P)H-Oxidase wieder zu NAD(P)⁺ oxidiert wird, wonach die Enzyme entfernt werden, **dadurch gekennzeichnet, dass** eine H₂O-bildende NAD(P)H-Oxidase eingesetzt wird, welche eine Aminosäuresequenz aufweist, die ausgewählt ist aus der Gruppe bestehend aus:
i) einer Aminosäuresequenz, die eine Identität zu SEQ ID Nr. 8 oder SEQ ID Nr. 10 von mindestens 80% aufweist,
ii) einer Aminosäuresequenz, die durch eine Nukleinsäure kodiert wird, die eine Identität zu SEQ ID Nr. 7 oder SEQ ID Nr. 9 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 7 oder SEQ ID Nr. 9 bindet.

2. Verfahren nach Anspruch 1, **dadurch gekennzeichnet, dass** die NAD(P)⁺-abhängige Oxidoreduktase zur Oxidation von 5-Formyl-2-furancarbonsäure zu 2,5-Furandicarbonsäure eine Aldehyd-Dehydrogenase ist.

3. Verfahren nach einem der Ansprüche 1 oder 2, **dadurch gekennzeichnet, dass** die NAD(P)⁺-abhängige Oxidoreduktase zur Oxidation von 5-Formyl-2-furancarbonsäure zu 2,5-Furandicarbonsäure eine Aminosäuresequenz aufweist, welche ausgewählt ist aus der Gruppe bestehend aus:
i) einer Aminosäuresequenz, die eine Identität zu SEQ ID Nr. 2, SEQ ID Nr. 4 oder SEQ ID Nr. 6 von mindestens 80% aufweist,
ii) einer Aminosäuresequenz, die durch eine Nukleinsäure kodiert wird, die eine Identität zu SEQ ID Nr. 1, SEQ ID Nr. 3 oder SEQ ID Nr. 5 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 1, SEQ ID Nr. 3 oder SEQ ID Nr. 5 bindet.
